# EUROPÄISCHE PATENTSCHRIFT

(11) **EP 0 582 976 B1**
(45) Veröffentlichungstag und Bekanntmachung des Hinweises auf die Patenterteilung: **09.02.2000**
(21) Anmeldenummer: 93112617.1
(22) Anmeldetag: 06.08.1993
(51) Int. Cl.: C12N 9/22

(54) **Typ-II-Restriktionsendonuklease SexAI**
Type II-restriction endonuclease sexAI
Type II endonucléase de restriction, sexAI

(30) Priorität: 12.08.1992 DE 4226657
(43) Veröffentlichungstag der Anmeldung: 16.02.1994
(73) Patentinhaber: Roche Diagnostics GmbH, 68305 Mannheim (DE)
(72) Erfinder: Kaluza, Klaus, Dr., D-83670 Bad Heilbrunn (DE); Auer, Johannes, Dr., D-82377 Penzberg (DE); Frey, Bruno, Dr., D-82377 Penzberg (DE)

(56) Entgegenhaltungen:
- EP-A- 0 413 958
- EP-A- 0 480 343
- DE-A- 4 010 108
- NUCLEIC ACIDS RESEARCH, Bd.18, Nr.SUP., 1990, ARLINGTON, VIRGINIA US Seiten 2331 - 2365 ROBERTS R.J. 'Restriction enzymes and their isoschizomers'

## Beschreibung

Die Erfindung betrifft die neue Typ II-Restriktionsendonuklease SexAI, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht immer noch Bedarf an der Schaffung weiterer Typ II-Restriktionsendonukleasen, die für DNA-Sequenzen spezifisch sind und die bisher von keiner der bekannten Restriktionsendonukleasen erkannt werden. Der Erfindung liegt daher die Aufgabe zu Grunde, eine neue Restriktionsendonuklease zur Verfügung zu stellen, welche eine bisher von keinem derartigen Enzym erkannte Sequenz spezifisch zu erkennen und zu spalten vermag.

Gelöst wird diese Aufgabe erfindungsgemäß durch eine Typ-II-Restriktionsendonuklease mit der Erkennungssequenz und der durch die Markierung definierten Spaltstelle

        A
5'-A | CC GG T-3'
        T
        A
3'-T GG CC | A-5'
        T

Die erfindungsgemäße neue Restriktionsendonuklease, die im folgenden als SexAI bezeichnet wird, hat ein Temperaturoptimum bei 37°C. Das Enzym besitzt eine gute Aktivität zwischen pH 8,2 und pH 9,5 in 10 mmol/l Tris/HCl-Puffer mit 1,0 mmol/l 2-Mercaptoethanol, 5 mmol/l MgCl₂ und 100 mmol/l NaCl. Das pH-Optimum liegt bei pH 9.2.

Die Erkennungssequenz läßt sich durch vollständigen Verdau der DNAs der Viren SV4O und Adeno 2, der Phagen Lambda (dcm⁻) und phiX174 bestätigen. Diese DNA-Moleküle werden mit SexAI behandelt.

Tabelle 1 zeigt einen Vergleich der experimentell beobachteten Schnittstellenspezifität mit einer computerermittelten Schnittstellenspezifität für ein Enzym, welches folgende Sequenz erkennt:

      A
5'-ACC GGT-3'
      T

**Tabelle I**

| DNA | Anzahl der experimentell bestimmten Schnittstellen | Anzahl der durch Computeranalyse bestimmte Schnittstellen | Experimentell bestimmte Fragmentlängen (Basenpaare) | | Durch Computeranalyse bestimmte Fragmentlängen (Basenpaare) | | Spaltpositionen ermittelt durch Computeranalyse (bei Basenpaar) | | |
|---|---|---|---|---|---|---|---|---|---|
| SV4O | 1 | 1 | 5243 | | 5243 | | 176 | | |
| PhiX174 | 1 | 1 | 5386 | | 5386 | | 3499 | | |
| Lambda | 5 | 5 | 22000 | 8700 | 22263 | 8745 | 22263 | 31008 | 32837 |
| | | | 7700 | 4100 | 7659 | 4095 | 40496 | 44407 | |
| | | | 3900 | 1800 | 3911 | 1829 | | | |
| Ad2 | 9 | 9 | 14000 | 4000 | 13758 | 3917 | 3356 | 5658 | 6399 |
| | | | 3400 | 3400 | 3369 | 3356 | 10316 | 11502 | 25260 |
| | | | 3050 | 2400 | 3074 | 2389 | 28334 | 31703 | 34092 |
| | | | 2300 | 1850 | 2302 | 1845 | | | |
| | | | 1200 | 750 | 1186 | 741 | | | |

Die Spaltposition innerhalb der Erkennungssequenz des Enzyms läßt sich an einem M13-Derivat, das im Abstand von ca. 30 - 200 Basen zur Bindungsstelle des universalen Sequenzierprimers (Messing, J. et al. (1981) Nucl. Acids Res. 9, 309 - 321) diese Erkennungssequenz trägt, bestimmen. An einzelsträngiger DNA des M13-Derivates werden zunächst mit dem universalen Sequenzierprimer Sequenzreaktionen nach der Didesoxy-Kettenabbruchmethode durchgeführt (Sanger, F., et al. (1977) Proc. Natl. Acad. Sci. USA 74, 560 - 564, Messing, J. et al. (1981) Nucl. Acids Res 9, 309 - 321).

Parallel dazu wird der Sequenzierprimer mit T4-Polynukleotid-Kinase und [γ-³²P]ATP am 5'-Ende radioaktiv markiert. Nach Anhybridisieren dieses 5'-endmarkierten Sequenzierprimers an die einzelsträngige M13-DNA wird in einer Auffüllreaktion mit DNA Polymerase I, (Klenow Enzym) und einer Desoxynukleotidtriphosphatmischung aus dATP, dCTP, dGTP und dTTP eine partiell doppelsträngige DNA hergestellt. Diese DNA, deren neusynthetisierter Strang am 5'-Ende radioaktiv markiert ist, wird mit der Restriktionsendonuklease SexAI gespalten. Die Hälfte des Spaltansatzes wird zusätzlich noch mit T4 DNA Polymerase in Gegenwart einer Mischung aller vier Desoxynukleotidtriphosphate behandelt, um glatte DNA-Enden zu erhalten.

Die Analyse der Reaktionsprodukte erfolgt durch Elektrophorese auf Sequenziergelen (8 mol/l Harnstoff, 5 % Polyacrylamid) und anschließender Autoradiographie. Die Interpretation der Ergebnisse wird nach Brown, N.L. und Smith, M. (Methods in Enzymology 65 (1980) 391 - 401) durchgeführt. Durch Vergleich der Laufstrecken der radioaktiv markierten Fragmente mit der Sequenzleiter wird die Lage der Spaltstelle bestimmt. Die zusätzlich mit T4 DNA Polymerase behandelten Proben zeigen im Vergleich zu der lediglich mit SexAI gespaltenen Probe eine um 5bp verlängerte Laufstrecke der Banden. Damit ist gezeigt, daß SexAI ein um 5bp 5'-überhängendes DNS-Ende erzeugt. Die Spaltung von SexAI erfolgt innerhalb der Erkennungssequenz daher mit folgender Spezifität:

        A
5'-A CC  GG T-3'
        T
        A
3'-T GG  CC A-5'
        T

Die experimentell bestimmte Anzahl der Schnittstellen ist identisch mit der Anzahl der Schnittstellen, die durch Computeranalyse mit den verschiedenen DNAs für die Sequenz

    A
5'-ACC GGT-3'
    T

erhalten wurde (Tabelle I). Zusätzlich wurden diese Daten noch mit den Tabellen aus Gene 1O (199O) 357 - 37O, verglichen.

Vorzugsweise wird SexAI gewonnen, indem man Mikroorganismen, vorzugsweise Streptomyceten, züchtet und das Enzym aus den Zellen gewinnt. Besonders bevorzugt wird der Stamm Streptomyces exfoliatus verwendet.

Der Mikroorganismus Streptomyces exfoliatus ist bei der Deutschen Sammlung von Mikroorganismen (DSM), Mascheroder Weg 1b, 33OO Braunschweig, BRD, hinterlegt und trägt die Hinterlegungsnummer DSM 7194.

Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda (dcm⁻)-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Die für die Gewinnung des Enzyms verwendeten Mikroorganismen wachsen aerob in LB-Medium (10 g/l Bacto-Trypton, 5 g/l Bacto-Yeast-Extrakt und 10 g/l NaCl). Die optimalen Wachstumsbedingungen sind bei 26°C, pH 6,5 - 7,5. Die Verdoppelungszeit beträgt etwa 2,5 Stunden.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen über eine French-Presse aufgeschlossen. Die weitere Reinigung des Überstands wird vorzugsweise über Affinitätschromatographie, und Adsorptionschromatographie durchgeführt. Als Material für die Affinitätschromatographie geeignet ist beispielsweise Heparin-Sepharose CL-6B (Pharmacia) und Fractogel TSK AF Orange (Merck). Ein geeigneter Adsorptionsaustauscher ist beispielsweise HA-Ultrogel (IBF).

Die folgenden Beispiele erläutern die Erfindung weiter.

### Beispiel 1

Der Mikroorganismus Streptomyces exfoliatus wird bei 26°C 10-12 Stunden gezüchtet und am Ende der logarithmischen Phase geerntet. Als Kulturmedium wird LB-Medium verwendet.

Die Zellpaste (3O g Naßgewicht) wird in 2,4 Volumen Puffer A (4O mmol/l Tris-HCl, pH 8,O, O,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.OOO lb/inch² aufgeschlossen und der Niederschlag abgetrennt. Der überstand wird mit NH₄Cl versetzt (Endkonzentration 0,3 mol/l). Durch eine Polymin-Fällung werden die Nucleinsäuren entfernt. Anschließend wird der abzentrifugierte überstand gegen Puffer B (40 mmol/l Tris-HCl, pH 8,0; 0,1 mmol/l EDTA; 7 mmol/l 2-Mercaptoethanol; 10% (w/v) Glycerin) dialysiert und über eine Heparin-Sepharose-Säule fraktioniert. Zur Elution wird ein Gradient von O,3 - 1 mol/l NaCl verwendet. SexAI wird in den Fraktionen zwischen O,4 und O,7 mol/l NaCl gefunden. Die aktiven Fraktionen werden gegen Puffer C (10 mmol/l Kaliumphosphat, pH 8,0; 0,1 mmol/l EDTA; 7 mmol/l 2-Mercaptoethanol; 10% (w/v) Glycerin) äquilibriert und auf einer HA-Ultrogel-Säule fraktioniert. Zur Elution wird ein Gradient von 10 - 250 mmol/l Kaliumphosphate-im Puffer C verwendet. Die aktiven Fraktionen werden gegen Puffer B dialysiert.

Anschließend werden sie auf eine Fraktogel TSK AF Orange-Säule, welche mit Puffer B äquilibriert wurde, aufgegeben. Zur Elution wird ein Gradient von 0,4 - 3 mol/l NaCl und 0,2 % Thesit in Puffer B verwendet.

Die aktiven Fraktionen werden zusammengegeben und gegen Lagerpuffer (2O mmol/l Tris-HCl, pH 8,O, 1O mmol/l 2-Mercaptoethanol, 1OO mmol/l NaCl, 0,1 mmol/l EDTA und 5O% (v/v) Glycerin) dialysiert.

### Beispiel 2

### Bestimmung der Aktivität

Definition der Enzymeinheiten: 1 U SexAI spaltet 1 µg Lamda dcm⁻¹ DNA innerhalb 1 Stunde bei 37°C in 25 µl Endvolumen.

Zu einer Mischung von 2,5 µl Inkubationspuffer (100 mmol/l Tris-HCl, pH 8,0, 37°C, 5O mmol/l Magnesiumchlorid, 1 mol/l NaCl und 10 mmol/l 2-ME) werden 17,9 µl Wasser und 3,6 µl Lambda dcm⁻ DNA (optische Dichte: 5,6 OD/ml) sowie 1 µl SexAI-Lösung (1 U/µl) zugegeben. Die Lösung wird eine Stunde bei 37°C inkubiert, auf Eis gekühlt und mit 5 µl eines Abstopp-Reagenses, bestehend aus 7 mol/l Harnstoff, 2O % (w/v) Saccharose, 6O mmol/l EDTA und O,O1 % (w/v) Bromphenolblau versetzt. Anschließend wird eine Trennung durch Elektrophorese in 0,5 % Agarose-Gelen für 3 - 4 Stunden bei 1OO V durchgeführt. Die erhaltenen Banden werden über Vergleich mit einem DNA-Längen-standard identifiziert.

## Patentansprüche

1. Typ II-Restriktionsendonuklease mit der Erkennungssequenz und der durch die Markierung charakterisierten Schnittstelle 
        A
5'-A CC  GG T-3'
        T
        A
3'-T GG  CC A-5'
        T
 erhältlich aus Mikroorganismen der Gattung Streptomyces.

2. Restriktionsendonuklease nach Anspruch 1 dadurch gekennzeichnet, daß sie aus dem Mikroorganismus Streptomyces exfoliatus DSM 7194 erhältlich ist.

3. Restriktionsendonuklease nach den Anspruchen 1 oder 2, gekennzeichnet durch ein Temperatur-Optimum bei 37°C und ein pH-Optimum bei 9,2.

4. Verfahren zur Gewinnung einer TypII-Restriktionsendonuklease mit der Erkennungssequenz und der durch die Markierung charakterisierten Spaltstelle 
       A
5'-A|CC GG T-3'
       T
       A
3'-T GG CC|A-5'
       T
 dadurch gekennzeichnet, daß man Streptomyceten züchtet und das Enzym aus den Zellen gewinnt.

5. Verfahren nach Anspruch 4, dadurch gekennzeichnet, daß man den Stamm Streptomyces exfoliatus DSM 7194 züchtet.

6. Verfahren nach den Anspruchen 4 und 5, dadurch gekennzeichnet, daß man die Zellen aufschließt und den Zellüberstand gewinnt.

7. Verfahren nach Anspruch 6, dadurch gekennzeichnet, daß man zur Feinreinigung den Zellüberstand Affinitatschromatograpnien und einer Adsorptionschromatographie unterwirft.

8. Verfahren nach Anspruch 7, dadurch gekennzeichnet, daß man zur Affinitätschromatographie trägerfixiertes Heparin verwendet.

9. Verwendung einer TypII-Restriktionsendonuklease mit der Erkennunqssequenz und der durch die Markierung charakterisierten Schnittstelle 
       A
5'-A|CC GG T-3'
       T

       A
3'-T GG CC|A-5'
       T
 erhältlich aus Mikroorganismen der Gattung Streptomyces zur Erkennung und Spaltung der komplementaren doppel-strangigen DNA-Sequenz

      A
5'-ACC GGT-3'
      T

## Claims

1. Type II restriction endonuclease having the recognition sequence 
      A
5'-A CC GG T-3'
      T
      A
3'-T GG CC A-5'
      T
 and the cleavage site characterized by the mark, obtainable from microorganisms of the genus Streptomyces.

2. Restriction endonuclease as claimed in claim 1, wherein it is obtainable from the microorganism Streptomyces exfoliatus DSM 7194.

3. Restriction endonuclease as claimed in claim 1 or 2, characterized by a temperature optimum at 37°C and a pH optimum at 9.2.

4. Process for the isolation of a type II restriction endonuclease having the recognition sequence 
      A
5'-A CC GG T-3'
      T
      A
3'-T GG CC A-5'
      T
 and the cleavage site characterized by the mark, wherein Streptomyces are cultured and the enzyme is isolated from the cells.

5. Process as claimed in claim 4, wherein the strain Streptomyces exfoliatus DSM 7194 is cultured.

6. Process as claimed in claims 4 and 5, wherein the cells are lysed and the cell supernatant is isolated.

7. Process as claimed in claim 6, wherein for the fine purification, the cell supernatant is subjected to affinity chromatographies and an adsorption chromatography.

8. Process as claimed in claim 7, wherein carrier-bound heparin is used for the affinity chromatography.

9. Use of a type II restriction endonuclease having the recognition sequence 
        A
5'-A CC GG T-3'
        T
        A
3'-T GG CC A-5'
        T
 and the cleavage site characterized by the mark obtainable from microorganisms of the genus Streptomyces for the recognition and cleavage of the complementary double-stranded DNA sequence

A
5'-ACC GGT-3'
T

## Revendications

1. Type II endonucléase de restriction possédant la séquence de reconnaissance et le site de clivage caractérisé par le marquage 
5'-A CC^A_TGG T-3'
3'-T GG^A_TCC A-5'
 et pouvant être obtenue à partir de micro-organismes du genre Streptomyces.

2. Endonucléase de restriction selon la revendication 1, caractérisée en ce qu'elle peut être obtenue à partir du micro-organisme Streptomyces exfoliatus DSM 7194.

3. Endonucléase de restriction selon les revendications 1 ou 2, caractérisée par une température optimum de 37°C et un pH optimum de 9,2.

4. Procédé de production d'une endonucléase de restriction de type II possédant la séquence de reconnaissance et le site de clivage caractérisé par le marquage 
        A
5'-A CC   GG T-3'
        T

        A
3'-T GG   CC A-5'
        T
 caractérisé en ce qu'on cultive des Streptomyces et extrait l'enzyme à partir des cellules.

5. Procédé selon la revendication 4, caractérisé en ce qu'on cultive la souche Streptomyces exfoliatus DSM 7194.

6. Procédé selon les revendications 4 et 5, caractérisé en ce qu on éclate les cellules et récupère le surnageant résultant.

7. Procédé selon la revendication 6, caractérisé en ce qu'on soumet le surnageant à des chromatographies d'affinité et à une chromatographie d'adsorption pour réaliser une purification fine.

8. Procédé selon la revendication 7, caractérisé en ce qu'on utilise pour la chromatographie d'affinité de l'héparine fixée sur support.

9. Utilisation d'une endonucléase de restriction de type II possédant la séquence de reconnaissance et le site de clivage caractérisé par le marquage 
        A
5 '–A CC  GG T-3 '
         T

        A
3 '–T GG  CC A-5 '
         T
 et pouvant être obtenue à partir de micro-organismes du genre Streptomyces pour reconnaître et couper la séquence d'ADN complémentaire à deux brins
5'-A CC^{A}_{T}GGT-3'
